Europäisches Patentamt

(19) European Patent Office

Office européen des brevets

(11) EP 0 947 941 B1

(12) EUROPEAN PATENT SPECIFICATION

(45) Date of publication and mention
of the grant of the patent:
21.05.2003 Bulletin 2003/21

(51) Int Cl.7: G06F 19/00, G06F 17/00

(21) Application number: 99105772.0

(22) Date of filing: 22.03.1999

(54) **Devices for in-vivo determination of the compliance function and the systemic blood flow of a living being**

Vorrichtungen zum invivo Bestimmen des Nachgiebigkeitsfunktion und des körperlichen Blutkreislauf eines lebendes Wesens

Dispositifs pour le détermination in vivo du function de compliance et de la circulation sanguine physique d'un être vivant

(84) Designated Contracting States:
DE ES FR GB IT NL SE

(30) Priority: 31.03.1998 DE 19814371

(43) Date of publication of application:
06.10.1999 Bulletin 1999/40

(73) Proprietor: Pulsion Medical Systems AG
81829 München (DE)

(72) Inventors:
• Joeken, Stephan, Dr.,
c/o Pulsion Verwaltungs GmbH
81675 München (DE)
• Fähle, Matthias, Dr.,
c/o Pulsion Verwaltungs GmbH
81675 München (DE)
• Pfeiffer, Ulrich, Priv. Doz. Dr. med.
81667 München (DE)

(74) Representative: Kehl, Günther, Dipl.-Phys. et al
Patentanwaltskanzlei
Günther Kehl
Friedrich-Herschel-Strasse 9
81679 München (DE)

(56) References cited:
• CAPPELLO A ET AL: "ANALYSIS OF THE ARTERIAL PRESSURE-VOLUME CURVE IN THE THREE-ELEMENTWINDKESSEL MODEL" PROCEEDINGS OF THE COMPUTERS IN CARDIOLOGY CONFERENCE,US,LOS ALAMITOS, IEEE COMP. SOC. PRESS, vol. -, 5 September 1993 (1993-09-05), pages 385-388, XP000478332 ISBN: 0-8186-5470-8
• J.K.J. LI ET AL: "A Nonlinear Model of the Arterial System Incorporating a Pressure Dependent Compliance" IEEE TRANSACTIONS ON BIOMEDICAL ENGINNERING, vol. 37, no. 7, July 1990 (1990-07), pages 673-678, XP002155957 USA
• FESKE W ET AL: "Vascular compliance after nitroprusside in hypertension" PROCEEDINGS OF THE FOURTEENTH ANNUAL NORTHEAST BIOENGINEERING CONFERENCE (IEEE CAT. NO.88-CH2666-6), DURHAM, NH, USA, 10-11 MARCH 1988, pages 277-280, XP002155958 1988, New York, NY, USA, IEEE, USA
• STERGIOPULOS N ET AL: "The four-element Windkessel model" PROCEEDINGS OF THE 18TH ANNUAL INTERNATIONAL CONFERENCE OF THE IEEE ENGINEERING IN MEDICINE AND BIOLOGY SOCIETY. BRIDGING DISCIPLINES FOR BIOMEDICINE' (CAT. NO.96CH36036), PROCEEDINGS OF 18TH ANNUAL INTERNATIONAL CONFERENCE OF THE IEEE ENGINEERING I, pages 1715-1716 vol.4, XP002155959 1997, New York, NY, USA, IEEE, USA ISBN: 0-7803-3811-1

**Description**

The invention relates to a device for individual in-vivo determination of the compliance function $C(p) = dV/dp$ of the vascular system downstream of a ventricle of a living being from the blood pressure $p(t)$ and a reference cardiac output COref.

The invention also relates to a device for continuously determining the systemic blood flow $q(t)$, in which the blood pressure $p(t)$ in the aorta or in the vicinity of the aorta is determined continuously.

Methods and devices of the type mentioned above are known. In the past, a plurality of methods have been developed with the purpose of calculating the cardiac output (CO) from the arterial blood pressure. On the one hand, methods are proposed in which the CO is determined from a few characteristic values such as, for example, mean arterial pressure (MAP), systolic and diastolic pressure (APsys, APdia), ejection time (ET) and patient data (age, sex, weight, height) [4,6,7], and on the other hand algorithms are used in which the complete contour of the pulsating blood pressure curve is utilized to calculate the cardiac output [1,5,20]. In the latter methods, which are also referred to as pulse contour analysis, two different approaches have so far been adopted. Firstly, the CO has been calculated directly from the arterial blood pressure with the aid of some correction factors [19,20] while in other work [5,25] a blood flow is calculated from the pressure, together with particular assumptions, and is assumed to correspond to the actual blood flow in the aorta and therefore to be usable for determining the cardiac output.

The classical Windkessel model, which was first proposed by Hales [26] and has been used by Frank [27] to determine the stroke volume (SV) and, together with the heart rate, the cardiac output, uses only the peripheral resistance R and the compliance C for modeling the cardiovascular system in question. In this model, the arterial blood flow is described by $q(t)$, which can be calculated for given C and R with the aid of the blood pressure $p(t)$ which is to be measured. However, closer examination shows that this simple model reproduces the physiological conditions only incompletely, with the result that many modifications to the original model have been proposed; for an overview reference may be made to [24,28].

The accuracy of these methods depends essentially on how well the assumptions, i.e. the model used, reflect the conditions in the cardiovascular system in question, and in [5] a nonlinear Windkessel model is thus used whose parameters are dictated by the age and sex of the patient. More recent investigations [21] show, however, that the model used in [5] does not reproduce the correct underlying physiological conditions; in particular the compliance (extensibility) of the vessels cannot always be described by the compliance/pressure relationship given in [5]. There may be several causes for this discrepancy. First, only a dependence of the in-vitro determined aortic cross section on the blood pressure is taken into account in [5] and a length variation, as described in [22,23] is neglected; also, only the density of the blood and not the strongly hematocrit-dependent viscosity is taken into account, and the compliance of the peripheral system is likewise ignored. Secondly, apart from age and sex, the compliance function $C(p)$ of a particular individual cannot be used in the method described in [5]. However, it is precisely in the examination of pathophysiological cases, e.g. arteriosclerosis, that it cannot be assumed that $C(p)$ varies according to age and sex, so that the basic model describes the physiological conditions only incompletely [25]. Lastly, it has been shown in [24] that it is to be expected that a modified Windkessel model can reproduce underlying physiological conditions more precisely.

However, a common factor in all the models described above is that the model parameters, after they have been determined once, no longer depend on the condition of the cardiovascular system in question. Nevertheless, almost all parameters can change with time, and for example the systemic resistance R can change as a result of medication. Other parameters, including the expandability and length of the aorta, change so greatly with pressure that they actually have to be regarded as variable even within one heartbeat.

The fact that aortic impedance and compliance cannot be assumed to be constant has been shown both in animal experiments [22] and for humans [29]. Primary causes of this are the expandability, length variation and volume variation of the aorta and vessels in proximity to the aorta. The typical variation in the aortic volume V as a function of pressure has been described inter alia in [30]. Since the compliance of the system is given by

$$C(p) = \frac{dV}{dp} \qquad (4)$$

and because of the limited volume the compliance must tend toward zero for very high pressures and cannot be constant. Since the change in volume is caused by length and cross-sectional changes in the vessels, there is also a change in the aortic impedance which, according to the Navier-Stokes equation, is determined on the one hand by the cross section and density of the liquid and, on the other hand, by length, viscosity and density of the blood.

Pressure-dependent aortic impedance and compliance have been discussed inter alia in [5,21] and used therein to investigate nonlinear Windkessel models. In [5] it is in particular assumed that $C(p)$ can be established by age and sex of the patient. The impedance $Z(p)$ is also ignored in this approach. What is more, it follows from the results obtained in [21] that the model used in [5] may to some extent conflict with the true physiological situation. One cause of this is that the compliance and aortic impedance are preset. An approach of this type is unsuitable for taking into account the features characteristic of the patient in question. In addition, the method proposed in [5] cannot be applied without modifications to other species. Further, only the typical aortic diameter investigated beforehand in-vitro and the density of the blood are taken into account in [5]. The effect of aortic length variations, and the dynamic behavior of the vessels in proximity to the aorta and the peripheral vessels and the viscosity of the blood are ignored in the modeling of the conditions existing in vivo.

There is consequently not yet any method which, for individual in-vivo determination of the compliance/pressure relationship, employs the measured variables used here.

These disadvantages are to be eliminated by the device of the present invention by determining all model parameters of interest from measurements on the physiological system in question, i.e. human or animal. To this end, in particular, the blood pressure p(t) in the aorta or in proximity to the aorta is to be measured continuously and a reference cardiac output (COref) is to be measured at least one time. With the aid of these values, all the parameters can be established and then used for hemodynamic investigation.

The object of the invention is to provide a device for individual in-vivo determination of the compliance function of a living being, which reproduces physiological conditions as faithfully as possible.

A further object of the invention is to provide a device for continuously determining the systemic blood flow of a patient which has a low degree of invasiveness and describes the actual blood flow at any given time as accurately as possible.

The way in which these objects are achieved is described in the independent patent claims. Advantageous refinements can be found in the dependent patent claims.

According to the invention, a so-called Windkessel model is used whose parameters can be identified with the aid of a reference measurement in vivo. Subsequently, the systemic flow and other hemodynamic parameters are thereby determined. A Windkessel model adapted and modified in this way describes the cardiovascular system of the individual in question more accurately and can therefore be used for likewise more accurate calculation of the systemic flow and hemodynamic parameters derived therefrom. The method can also be applied directly to other species, without the need to determine previously hypothetical characteristics for this. The extra outlay for the newly developed method consists in the fact that, for calculating an individual compliance function, besides the continuous blood pressure measurement, the cardiac output has at least one time to be determined using a different method, e. g. arterial thermodilution.

The invention will be carried out according to attached independent claim 1.

The invention will be explained in more detail below with reference to an illustrative embodiment schematically represented in the figures, in which:

Figure 1     shows a preferred electrical model circuit for simulating the cardiovascular system in question.

Figure 2     shows a flow chart for calculating the aortic impedance Z(p), the compliance function C(p) and the blood flow q(t).

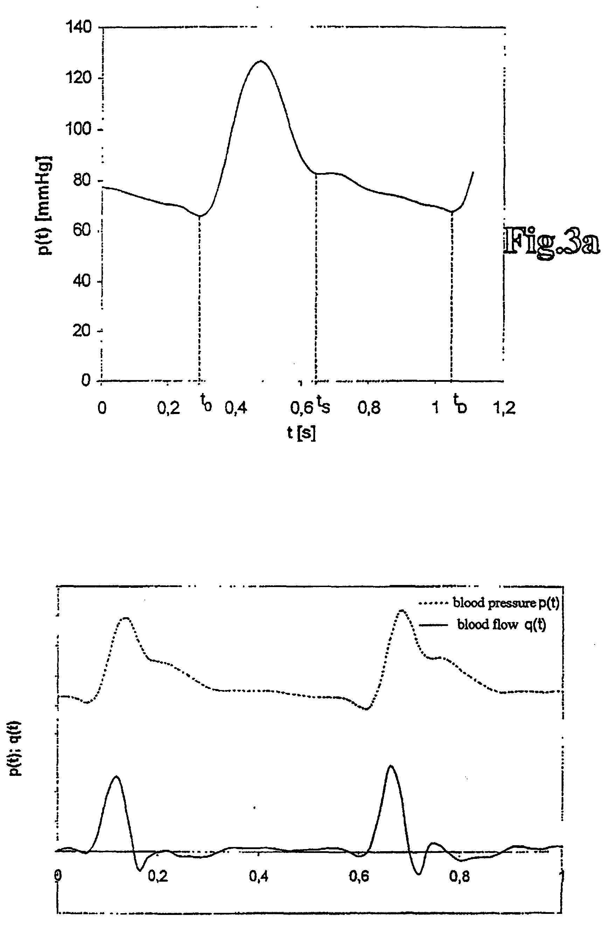

Figure 3a    shows a graph to illustrate the time dependence of the blood pressure p(t), $t_0$ denoting the time at which the aortic valve opens, ts the time at which the aortic valve shuts and tD the end time of the diastole.

Figure 3b    shows a graph to explain the time dependence of the blood pressure p(t) and the resulting blood flow q(t).

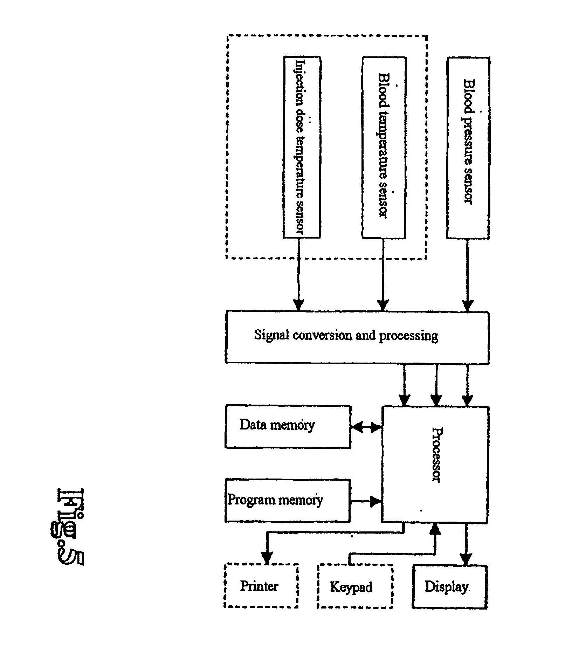

Figure 4     shows a typical compliance function C(p) of a human aorta.

Figure 5     shows a block circuit diagram of a device according to the invention.

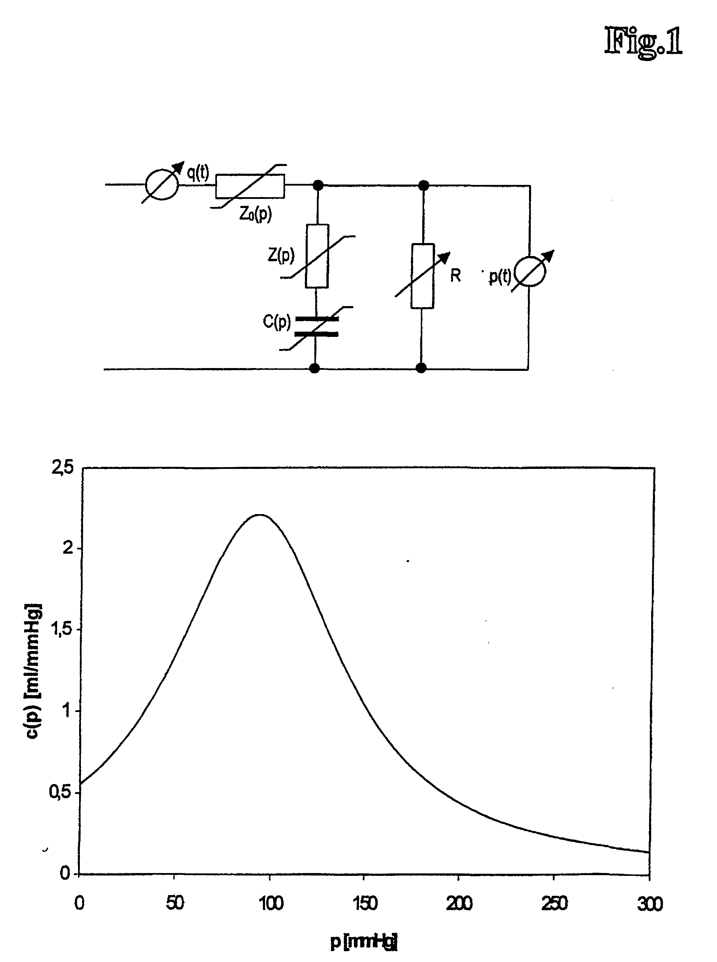

Figure 1 shows a nonlinear modified Windkessel model which is preferably used according to the invention, in which the aortic impedance functions Z(p) and Zo(p), the compliance function C(p) and the systemic resistance R are taken into account.

The resistor R in Figure 1 represents the time-variable peripheral flow resistance of the body. Zo(p) and Z(p) are nonlinear impedances which are dependent on the pressure p(t) and, together with the nonlinear pressure-dependent capacitance C(p), are intended to simulate the behavior of the aorta and the blood vessels in proximity to the aorta.

The result obtained for the model outlined in Figure 1 with the Fourier transform

$$\tilde{p}(\omega) \;=\; \int_{-\infty}^{\infty} p(t)\exp(-i\omega t)\,dt$$

and the function $\tilde{q}(\omega)$, which is to be calculated similarly, for the aortic impedance is

$$\lim_{\omega \to \infty} \frac{\tilde{q}(\omega)}{\tilde{p}(\omega)} = \frac{1}{R} + \frac{1}{Z} \qquad , \qquad (1)$$

so that for R > > Z it follows that

$$Z = \lim_{\omega \to \infty} \frac{\tilde{p}(\omega)}{\tilde{q}(\omega)} \qquad (2)$$

The following equation is further satisfied for the compliance C

$$C(p) = \frac{q(t) - p(t)/R}{\dot{p}(t) - Z(p) \cdot (\dot{q}(t) - \dot{p}(t)/R)} \qquad (3)$$

in which $\dot{p}(t) = dp(t)/dt$ and $\dot{q}(t) = dq(t)/dt$ are the respective differentials of the pressure and flow with respect to time. Equations (1) to (3) show that C and Z can be calculated for the model in Figure 1 if the systemic flow q(t), the blood pressure p(t) and the systemic resistance R are known.

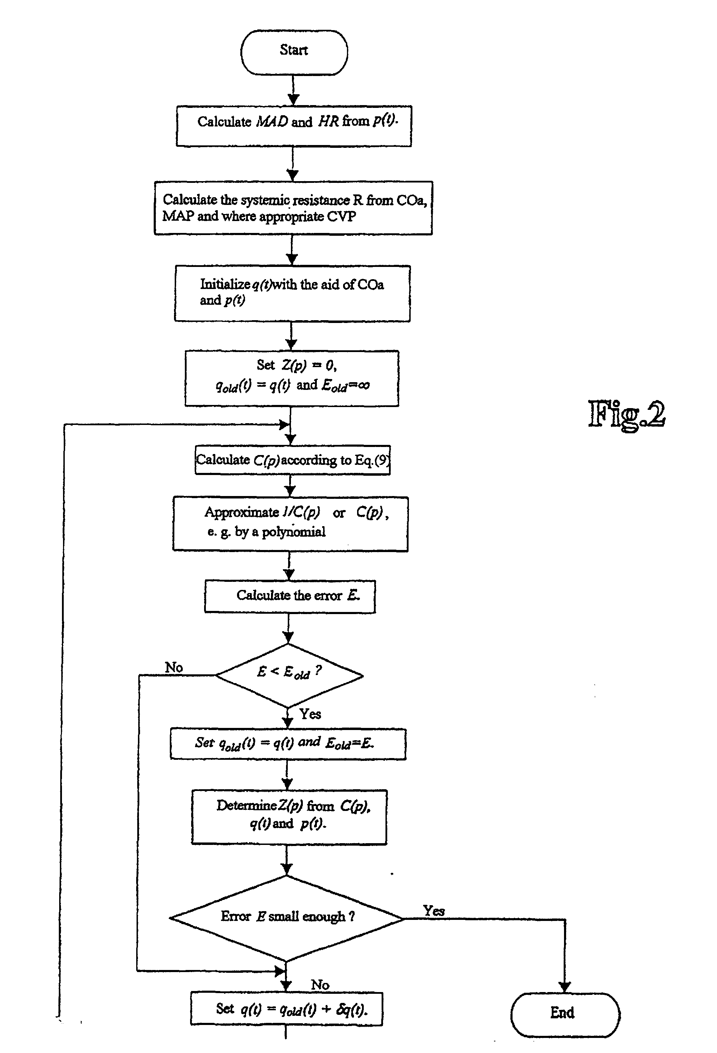

Figure 2 shows an overview of the refined method.

i) The mean arterial blood pressure MAP and the heart rate HR are firstly determined from the pressure p(t).

ii) Together with the reference cardiac output COref, which has preferably been determined by arterial thermodilution, and for which the following equation is satisfied

$$COref = HR \cdot \int q(t)dt \qquad (5)$$

the systemic resistance is calculated according to R=(MAP-CVP)/COref. In this expression, CVP is the central venous pressure which, if it is unknown, can be approximated by a suitable constant pressure, e.g. 0 mmHg.

iii) The next step is to establish a flow q(t), which should be chosen suitably, which is used as the start function in the subsequent iteration and satisfies the underlying physiological conditions. The blood flow q(t) describes the flow which passes directly from the left heart into the aorta. It is therefore required of q(t) that the subconditions

$$q(t0) = q(ts) = 0 \qquad \text{and} \qquad \int_{q(t)dt} = \frac{COref}{HR} = SV \qquad (6)$$

are satisfied for all heartbeats recorded during the measurement of the reference cardiac output COref. In this expression, the time t0 is the start of the systole and ts the end of the systole. The end of the diastole is denoted tD below. Suitable flows q(t) would be q(t) = 0 for $ts < t \leq t_D$ and for $t_0 < t \leq t_s$, for example

$$q(t) = \frac{\pi \cdot COref}{2 \cdot HR \cdot (t_s - t_0)} \sin\left(\frac{\pi}{t_s - t_0} (t - t_0)\right) \qquad (7)$$

or preferably

$$q(t) = \kappa[p(t) - (p(t_0) + (t - t_0)\frac{p(t_s) - p(t_0)}{t_s - t_0})] \qquad , \qquad (8)$$

in the latter case, $\kappa$ is to be determined from the condition $\int q(t)dt = COref/HR$. Besides the flows indicated in equations (7) and (8), however, other starting conditions are conceivable, for example a constant or parabolic flow.

iv) Set Z(p) = 0 and introduce the auxiliary variables qold(t) and Eold, which are initialized at qold(t)=q(t) and Eold

$= \infty.$

v) Calculate, from the blood pressure p(t) measured in the aorta or in proximity to the aorta, the flow q(t) and its time derivatives, the compliance function according to

$$C(p) = \frac{q(t) - p(t)/R}{\dot{p}(t) - Z(p) \cdot (\dot{q}(t) - \dot{p}(t)/R)} \tag{9}$$

vi) The inverse of the compliance function is then approximated by a polynomial of suitable order in p, i.e. in

$$\frac{1}{C(p)} = \sum_{k} \alpha_k p^k \tag{10}$$

the expansion coefficients are determined in such a way that the identity

$$q(t) = \frac{p(t)}{R} + \frac{1}{[\Sigma_k \alpha_k p^k]} \cdot [\dot{p}(t) \cdot Z(p) \cdot (\dot{q}(t) \cdot \dot{p}(t)/R)] \tag{11}$$

is optimally satisfied. As a suitable criterion for this, the mean square error

$$E = \left\langle \left( q(t) \cdot p(t)/R - [\dot{p}(t) \cdot Z(p) \cdot (\dot{q}(t) \cdot \dot{p}(t)/R)] / [\Sigma_k \alpha_k p^k] \right)^2 \right\rangle$$

may be minimized, it being possible to use p(t) and q(t) at all times or alternatively only from preferred time intervals. In this case and below, the notation <·> indicates calculation of the mean.

vii) If E < Eold, then set qold(t) = q(t) and Eold = E and continue with step viii), otherwise go to point x) .

viii) Calculate Z(p). On the one hand, the procedure adopted for this may be to determine Z(p) according to equation (1) or (2). In this case, it is assumed that these equations, which are initially valid only for the model in Fig. 1b in which the parameters are not pressure-dependent, also apply for sufficiently. short time intervals $\Delta t$ to the nonlinear approach proposed according to Figure 1. For the latter, the impedance function can then be ascertained according to equation (2) with

$$Z(p) = \int_{-\Delta t}^{+\Delta t} p(t)\, dt / 2\Delta t)$$

or, equally, by

$$Z(p) = \sqrt{(\int_{t-\Delta t}^{t+\Delta t} p^2(t)dt - (\int_{t-\Delta t}^{t+\Delta t} p(t)dt)^2)/(\int_{t-\Delta t}^{t+\Delta t} q^2(t)dt - (\int_{t-\Delta t}^{t+\Delta t} q(t)dt)^2)} \tag{12}$$

even directly from the time-dependent blood pressure p(t) and the blood flow q(t) without a prior Fourier transform. On the other hand, Z(p) can be calculated yet more simply by

$$Z(p) \neq \frac{A}{\sqrt{C(p)}} \tag{13}$$

as can be seen from the following discussion. The aortic diameter d and length l increase with rising pressure, so that to first approximation d I may be assumed. According to the Hagen-Poiseuille law, this results in $Z(p)\, \alpha\eta/V$, in which $\eta$ denotes the viscosity of the blood and V the aortic volume. With $C(p) = dV/dp$, this gives $C(p)\, \alpha\, d(1/Z)/dp$, from which equation (13) directly follows. The constant of proportionality A which it contains can, for example, be determined by determining the function $Z(p)$ for at least one pressure p according to equation (12).

ix) If the error E is small enough, then the identification of the model parameters is terminated here. Otherwise, it continues with x).

x) The assumed blood flow should finally be varied in such a way that the stroke volume further corresponds to the stroke volume $SV = COref/HR$ which follows from the reference cardiac output. Since, at this time, qold(t) always describes the optimum flow so far, $q(t)=qold(t) + \delta q(t)$ with $\int \dot{\delta q}(t)dt=0$ is set.

xi) Continue with step v).

The algorithm indicated in i)-xi) describes the preferred method, in which the reference cardiac output COref and the continuously measured arterial pulse curve p(t) are used to determine all-the other values. This guarantees that the compliance function and the aortic impedance function are determined in such a way as is required by the interactions actually taking place in the cardiovascular system in question. In particular, C(p) thereby takes into account not only the variation in the aortic cross section, but also the actual variation in volume of the aorta and peripheral vessels; likewise, a variation in the length of the aorta and the density and viscosity of the blood are described by $Z(p)$. With the aid of the description which is introduced in step vi) and can reproduce any physiologically feasible compliance/pressure relationship, it also becomes possible to extrapolate C(p) and Z(p), so that the functions can even be applied beyond the pressures observed during the calibration, i.e. during the measurement of the reference cardiac output.

The algorithm indicated describes the preferred method. Other methods can be readily obtained from it and are covered by the appended claims. In particular, in vi), instead of the inverse of the compliance function, it is also possible for C(p) to be described by a finite Taylor series, i.e. by a polynomial. It is also conceivable for steps viii) and ix) to be interchanged with each other, or for the criterion to be optimized to be modified in step vi). For example, instead of minimizing the mean square error, it is also possible for the expectation to be maximized.

In order to accelerate the method, it is in particular possible to configure the choice of the initial blood flow q(t) in step iii) in such a way that C(p) is initially determined only from the diastoli, i.e. for $ts < t \leq t_D$. According to equation (9),

$C(p) = - p(t) / R \cdot \dot{p}(t)$ is then satisfied for Z=0, so that for all $p(t) < p(ts)$ the compliance function can therefore be used to expand the flow during the systole in terms of an orthogonal function system, e.g.

$$q(t) = \sum_k q_k \sin(k \cdot \pi \cdot (t - t_0)/(t_s - t_0)) \tag{14}$$

The coefficients qk are in this case determined by minimizing the expression

$$\left\langle \left( (q(t) - p(t) / R - \dot{p}(t) / C(p) \right)^2 \right\rangle \tag{15}$$

the above error being to be calculated from all $p(t) < p(ts)$, including those which occur at the start of the systole. With this choice of the flow, if the cardiovascular system is in good enough condition, just one or a few iterations will be enough to calculate the model parameters using the described algorithm.

The method may be further accelerated in the case in which, in step vi), only pressure values from the diastole ranges are used and 1/C(p) is approximated by a second-degree polynomial. During the first iteration, the following result is obtained for this application

$$C(p) = \frac{MAP^2 \cdot COref}{\langle \dot{p}(t) \rangle} \cdot \frac{1}{3 \cdot MAP \cdot p - 3 \cdot MAP^2 \cdot p^2} \tag{16}$$

After the parameters of the model in Figure 1 have been determined from the blood pressure p(t) in the aorta or in proximity to the aorta and from the reference cardiac output COref, the cardiac output can subsequently be calculated continuously just from the blood pressure beat by beat. For this purpose, the start of the heartbeat, which is again denoted to below, needs to be calculated and equation (11) needs to be calculated for the initial condition $q(t_o) = \dot{q}(t_o)=0$. A calculation of this type can be carried out numerically, e.g. using Runge-Kutta, Euler or another generally known algorithm. If, further, the required blood pressure measurement is available only at discrete time intervals, then linearization can be carried out between them if required. For the stroke volume thus determined only using the blood pressure, the following is satisfied with $\gamma = 1$

$$SV = \gamma\int q(t)dt , \qquad (17)$$

so that the following is obtained for the continuously determined cardiac output CO

$$CO = HR{\cdot}SV = HR{\cdot}\gamma{\cdot}\int q(t)dt \qquad (18)$$

In the expression above, HR denotes the heart rate, which is likewise to be calculated from the pulse curve p(t) and indicates the number of beats per minute. The integration occurring in equations (16) and (17) can in this case be carried out over the entire heartbeat, or alternatively only over the length of the systole, since $q(t)=0$ is satisfied during the diastole. If the stroke volume SV, and therefore CO as well are calculated during the entire heartbeat, then it is not necessary to determine the end of the systole. To do this (see e.g. [31]), either accurate analysis of the pressure curve would be necessary, in order to determine the position of the so-called dichrotic notch and therefore the end of the systole from p(t), or further measuring instruments such as an ECG would be required. Integration over the entire period is consequently more robust and less involved than those methods which evaluate only a particular period of the heartbeat. If, in addition, the continuously determined cardiac output CO is also calculated from those blood pressure measurements which, together with the reference cardiac volume COref, have been used for model identification using the method described above, then the accuracy of the method for continuous CO calculation can be increased further in that CO=COref must be satisfied and the calibration factor $\gamma$ is therefore determined according to

$$\gamma = \frac{COref}{HR \cdot \int q(t)dt} \qquad (19)$$

In order to use the described method, it is necessary to have a device whose basic structure is represented in Figure 5. In this diagram, the components represented by a broken line are optional and at least some of them may be omitted in a minimum configuration of the device. A device of this type consists at least of an evaluation unit, usually a central processing unit, in which the method for determining the individual compliance function C(p) is implemented on its own or together with other methods; in particular, the method for continuously calculating the cardiac output may be employed in the same device. It is also necessary to have a sensor for measuring the blood pressure p(t) in the aorta or in proximity to the aorta and an arrangement for signal processing and signal conversion, a program memory and a data memory, as well as a device for providing the reference cardiac output COref. If COref is determined through arterial thermodilution, then this unit consists at least of a blood temperature sensor and a sensor for measuring the temperature of the injection dose used by this method, see [8]. Since, however, COref can also be obtained in other ways, this module may also have a different form or entry may take place through a keypad which may also be used in the device for the user to enter instructions. There will also be at least one of the options to have the results of the evaluation displayed, printed out or stored on a bulk storage device (not shown).

References:

[1]    U.S: Pat. 5 183 051; Kraidin et al.
[2]    U.S. Pat. 5 211 177; Chesney et al.
[3]    U.S. Pat. 5 241 966; Finkelstein et al.
[4]    U.S. Pat. 5 316 004; Chesney et al.
[5]    U.S. Pat. 5 400 793; Wesseling
[6]    U.S. Pat. 5 535 753; Petrucelli et al.
[7]    U.S. Pat. 5 647 369; Petrucelli et al.
[8]    U.S. Pat. 5 526 817; Pfeiffer et al.

[19]    Werner et al. (1953); J. Appl. Physiol. **5**:495

[20]  Wesseling et al. (1983);
       Adv. cardivasc. Phys. **5**:16-52.
[21]  Fogliari et al. (1996);
       Modeling in Physiol. **H**:2661- 2668
[22]  Gentile et al. (1988); Cardiovasc. Res. **22**:385-389
[23]  Wuyts et al. (1995); Phys. Med. Biol. **40**:1577-1597
[24]  Toy et al. (1985); IEEE Trans. Biomed. Eng.
       **32(2):**174-176
[25]  Richter et al. (1984); Biorheology **21**:723-734
[26]  Hales (1733); Statical Essays: Containing
       Haemostatics
[27]  Frank (1899); Z. Biol. **37**:483-526
[28]  Yoshigi et al. (1997); Am. J. Physiol. **273**
       (Heart Circ. Physiol. **42**):H19-H27
[29]  Langewouters et al. (1984);
       J. Biomechanics **17:**425-435
[30]  Hallock et al. (1937); J. Clin. Invest. **16:**597
[31]  Hoskels et al. (1997); J. Clin. Mon. **13:**309-316

**Claims**

1. A device for individual in-vivo determination of the compliance function C (p) = dV/dp of the vascular system downstream of a ventricle of a living being from the blood pressure p (t) and a reference cardiac output COref, wherein said device comprising:

   a) a pressure sensor means adapted to continuously determine the pressure p (t) in the aorta or in the vicinity of the aorta;
   b) computer means comprising

      b1) means adapted to calculate the mean blood pressure MAP from the continuously determined blood pressure p(t),
      b2) means adapted to calculate the systemic resistance R of the body as

$$R = \frac{MAP - CVP}{COref} \ ,$$

      CVP being an arbitrary central venous pressure which is ascertained or estimated, and COref being a reference value for the cardiac output,
      b3) means to calculate the first differential of the blood pressure with respect to time $\dot{p}(t) = dp/dt$ , and
      b4) means to calculate the compliance function C(p) at least from p(t), $\dot{p}(t)$ and R by means of a nonlinear modified Windkessel model.

2. The device as claimed in claim 1, wherein said values of p(t) are values satisfying the following condition for calculating the compliance function C(p):

$$p(t) \leq p(ts) \ ,$$

   ts being the time when the aortic valve shuts.

3. The device as claimed in claim 1, wherein only blood pressure values from the diastole are used in the calculation of the compliance function C(p).

4. The device as claimed in claim 1, wherein only blood pressure values from the systole are used in the calculation of the compliance function C(p).

5. The device as claimed in anyone of the preceding claims, wherein said computer further comprising means adapted to determine a blood flow q(t) on the basis of the pressure p(t) and the first time derivative dp/dt, and to calculate said compliance function according to

$$C(p) = \frac{q(t) - p(t)/R}{\dot{p}(t) - Z(p)\cdot(\dot{q}(t) - \dot{p}(t)/R)}$$

for arbitrary impedance functions Z(p) and arbitrary times t in such a way that

$$q(t) = \frac{p(t)}{R} + C(p)[\dot{p}(t) - Z(p)\cdot(\dot{q}(t) - \dot{p}(t)/R)]$$

is satisfied.

6. The device as claimed in anyone of the preceding claims, wherein said computer further comprising means adapted to approximate the inverse of the compliance function 1/C(p), by a finite-order polynomial and to extrapolate C(p) beyond the pressure range recorded when determining the reference cardiac output according to said polynomial.

7. The device as claimed in claim 1, wherein said computer further comprising means adapted to determine the minimum of the function

$$\sum \int_{t=t_0}^{t=t_s} \left( q(t) - \frac{p(t)}{R} - \frac{\dot{p}(t) - Z(p)\cdot(\dot{q}(t) - \dot{p}(t)/R)}{\sum_k \alpha_k p^k} \right)^2 dt$$

and to calculate the individual compliance function C(p) as

$$C(p) = \frac{1}{\sum_k \alpha_k p^k}.$$

8. The device as claimed in claims 3 and 6, wherein said computer further comprising means adapted to describe the inverse of the compliance function C(p) by a second-order polynomial and to approximate C(p) according to

$$C(p) = \frac{MAP^2 COref}{\langle \dot{p}(t) \rangle} \cdot \frac{1}{3\cdot MAP\cdot p - 3\cdot MAP^2 - p^2}$$

9. The device as claimed in anyone of the preceding claims, wherein said computer further comprising means adapted to approximate the compliance function C(p) by a finite-order polynomial and to extrapolate C(p) beyond the pressure range recorded when determining the reference cardiac output according to said polynomial.

10. The device as claimed in claim 1, wherein said computer further comprising means adapted to determine the minimum of the function

$$\sum \int_{t=t_0}^{t=t_s} \left( q(t) - \frac{p(t)}{R} - \left[ \sum_k \beta_k p^k \right] \cdot \left[ \dot{p}(t) - Z(p)\cdot(\dot{q}(t) - \dot{p}(t) - \dot{p}(t)/R) \right] \right)^2 dt$$

and to calculate the individual compliance function C(p) as

$$C(p) = \sum_{k} \beta_k p^k$$

11. The device as claimed in claim 3 and 5, wherein said computer further comprising means adapted to use compliance function C(p) calculated for p(t)≤p(ts) to expand the blood flow q(t) in terms of a complete function system and to describe q(t) in the form of a Fourier series according to the following equation

$$q(t) = \sum_{k} q_k \sin\left( k \cdot \pi \cdot \frac{t - t_0}{t_s - t_0} \right)$$

the coefficients qk being determined by minimizing the mean square error and the values to and ts denoting the times when the aortic valve is opened and shut.

12. The device as claimed in anyone of the preceding claims, wherein said computer further comprising means adapted to vary the assumed blood flow q(t) in such a way as to minimize the mean square error.

13. The device as claimed in anyone of the preceding claims, wherein said computer further comprising means adapted to determine the aortic impedance/pressure relationship according to

$$Z(p) = \frac{A}{\sqrt{C(p)}}$$

A being a constant of proportionality.

14. The device as claimed in anyone of the preceding claims, wherein said computer further comprising means adapted to determine a nonlinear aortic impedance function using the Fourier transforms of the blood pressure $\tilde{p}(\omega)$ and blood flow $\tilde{q}(\omega)$ according to

$$1/Z = \tilde{q}(\omega \to \infty)/\tilde{p}(\omega \to \infty) - 1/R$$

or

$$Z(p) = \tilde{p}(\omega \to \infty)/\tilde{q}(\omega \to \infty)$$

15. The device as claimed in claim 1, wherein said computer further comprising means adapted to determine a blood flow q(t) on the basis of the pressure p(t) and the first time derivative dp/dt and to calculate the impedance function

$$Z\left( p = \int_{-\Delta t}^{+\Delta t} p(t)\,dt / 2\Delta t \right)$$

according to

$$Z(p) = \sqrt{ \left( \int_{t-\Delta t}^{t+\Delta t} p^2(t)\,dt - \left( \int_{t-\Delta t}^{t+\Delta t} p(t)\,dt \right)^2 \right) \Big/ \left( \int_{t-\Delta t}^{t+\Delta t} q^2(t)\,dt - \left( \int_{t-\Delta t}^{t+\Delta t} q(t)\,dt \right)^2 \right) }$$

**16.** The device as claimed in anyone of the preceding claims, wherein said computer further comprising means adapted to approximate the aortic impedance function Z(p) by a finite-order polynomial and to extrapolate Z(p) beyond the pressure range recorded during calibration according to said polynomial.

**17.** A device as claimed in claim 1 further comprising means adapted to continuously determine the systemic blood flow q(t) of a living being, wherein said device further comprising computing means adapted to calculate said blood flow q(t) at least from $\dot{p}(t)$ p(t), and R using a nonlinear modified Windkessel model.

**18.** The device as claimed in claim 17, wherein said computing means further comprising means adapted to determine said systemic blood flow q(t) according to

$$q(t) = \frac{p(t)}{R} + C(p)\left[\dot{p}(t) - Z(p) \cdot \left(\dot{q}(t) - \dot{p}(t)/R\right)\right]$$

**19.** The device as claimed in claim 17, wherein said computing means further comprising means adapted to calculate the stroke volume SV by integrating the flow over a period of time in accordance with

$$SV = \int q(t)\,dt$$

and wherein said period of time corresponding to the heartbeat or the ejection time during the heartbeat.

**20.** The device as claimed in claims 1 and 17, wherein said computing means further comprising means adapted to calculate the stroke volume SV by comparing the continuous blood flow q(t) with a reference cardiac output COref according to

$$SV = \gamma \int q(t)\,dt \qquad \text{with} \qquad \gamma = \frac{COref}{HR \cdot \int q(t)\,dt}$$

**21.** The device as claimed in anyone of claims 16 and 17, wherein said computing means further comprising means adapted to calculate the stroke volume variation according to

$$SVV = \sqrt{\langle SV^2 \rangle - \langle SV \rangle^2}$$

and further comprising means adapted to correct the stroke volume value according to said stroke volume variation value taken alone or in combination with other parameters as mean blood pressure MAP, systolic pressure $AP_{SYS}$, diastolic pressure $AP_{DIA}$ and heart rate HR.

**22.** The device as claimed in anyone of the preceding claims, wherein said computing means further comprising means adapted to calculate the cardiac output continuously together with the heart rate HR from the stroke volume SV according to $CO = HR \cdot SV$.

**23.** The device as claimed in claim 22, wherein said computing means further comprising means adapted to determine the mean pressure MAP continuously from the blood pressure curve and to calculate the systemic resistance continuously according to

$$R = \frac{MAP - CVP}{CO}$$

for central venous pressure value CVP which has been measured or estimated.

**24.** The device as claimed in claim 22, wherein said computing means further comprising means adapted to determine the mean pressure MAP continuously from the blood pressure curve and to calculate the compliance function continuously according to

$$C(p) = \frac{MAP^2 CO}{\langle \dot{p}(t) \rangle} \cdot \frac{1}{3 \cdot MAP \cdot p - 3 \cdot MAP^2 - p^2}$$

## Patentansprüche

1.  Vorrichtung zur individuellen in-vivo Bestimmung der Compliance-Funktion C(p) = dV/dp des Gefäßsystems nach einer Herzkammer eines Lebewesens aus dem Blutdruck p(t) und einem Referenz-Herzzeitvolumen COref, wobei die Vorrichtung folgendes aufweist:

    a) Drucksensormittel, die angepaßt sind, den Druck p(t) in der Aorta oder in der Nähe der Aorta kontinuierlich zu bestimmen;
    b) Computermittel, die folgendes aufweisen:

    b1) Mittel, die angepaßt sind, den mittleren Blutdruck MAP aus dem kontinuierlich bestimmten Blutdruck p(t) zu berechnen,
    b2) Mittel, die angepaßt sind, den systemischen Widerstand R des Körpers wie folgt zu berechnen

    $$R = \frac{MAP - CVP}{COref} ,$$

    wobei CVP ein beliebiger zentralvenöser Druck ist, der ermittelt oder geschätzt wird, und COref ein Referenzwert für das Herzzeitvolumen ist,
    b3) Mittel zur Berechnung der ersten zeitlichen Ableitung des Blutdrucks nach der Zeit $\dot{p}(t)$ = dp/dt, und
    b4) Mittel zur Berechnung der Compliance-Funktion C(p) mindestens aus p(t), $\dot{p}(t)$ und R nach einem nichtlinearen modifizierten Windkessel-Modell.

2.  Vorrichtung nach Anspruch 1, bei der die Werte von p(t) Werte sind, die die folgende Bedingung zur Berechnung der Compliance-Funktion C(p) erfüllen:

    $$p(t) \le p(ts) ,$$

    wobei ts der Zeitpunkt des Schließens der Aortaklappe ist.

3.  Vorrichtung nach Anspruch 1, bei der nur Blutdruckwerte aus der Diastole zur Berechnung der Compliance-Funktion C(p) verwendet werden.

4.  Vorrichtung nach Anspruch 1, bei der nur Blutdruckwerte aus der Systole zur Berechnung der Compliance-Funktion C(p) verwendet werden.

5.  Vorrichtung nach irgendeinem der vorhergehenden Ansprüche, bei der der Computer ferner Mittel aufweist, die angepaßt sind, einen Blutfluß q(t) auf der Grundlage des Druckes p(t) und der ersten zeitlichen Ableitung dp/dt zu bestimmen und die Compliance-Funktion nach

    $$C(p) = \frac{q(t) - p(t)/R}{\dot{p}(t) - Z(p) \cdot (\dot{q}(t) - \dot{p}(t)/R)}$$

    für beliebige Impedanzfunktionen Z(p) und beliebige Zeiten t derart zu berechnen, dass

    $$q(t) = \frac{p(t)}{R} + C(p)[\dot{p}(t) - Z(p) \cdot (\dot{q}(t) - \dot{p}(t)/R)]$$

    erfüllt wird.

6.  Vorrichtung nach irgendeinem der vorhergehenden Ansprüche, bei der der Computer ferner Mittel aufweist, die angepaßt sind, den Kehrwert der Compliance-Funktion, 1/C(p) durch ein Polynom endlicher Ordnung zu approximieren und C(p) über den bei der Bestimmung des Referenz-Herzzeitvolumens aufgezeichneten Druckbereich hinaus gemäß diesem Polynom zu extrapolieren.

7. Vorrichtung nach Anspruch 1, bei der der Computer ferner Mittel aufweist, die angepaßt sind, das Minimum der Funktion

$$\sum \int_{t=t_0}^{t=t_s} \left( q(t) - \frac{p(t)}{R} - \frac{\dot{p}(t) - Z(p)\cdot(\dot{q}(t) - \dot{p}(t)/R)}{\sum_k \alpha_k p^k} \right)^2 dt$$

zu bestimmen und die individuelle Compliance-Funktion C(p) zu berechnen als

$$C(p) = \frac{1}{\sum_k \alpha_k p^k} \; .$$

8. Vorrichtung nach den Ansprüchen 3 und 6, bei der der Computer ferner Mittel aufweist, die angepaßt sind, den Kehrwert der Compliance-Funktion C(p) durch ein Polynom zweiter Ordnung zu beschreiben und C(p) wie folgt zu approximieren

$$C(p) = \frac{MAP^2 \, COref}{\langle \dot{p}(t) \rangle} \cdot \frac{1}{3\cdot MAP\cdot p - 3\cdot MAP^2 - p^2}$$

9. Vorrichtung nach irgendeinem der vorhergehenden Ansprüche, bei der der Computer ferner Mittel aufweist, die angepaßt sind, die Compliance-Funktion C(p) durch ein Polynom endlicher Ordnung zu approximieren und C(p) über den bei der Bestimmung des Referenz-Herzzeltvolumens aufgezeichneten Druckbereich hinaus gemäß diesem Polynom zu extrapolieren.

10. Vorrichtung nach Anspruch 1, bei der der Computer ferner Mittel aufweist, die angepaßt sind, das Minimum der Funktion

$$\sum \int_{t=t_0}^{t=t_s} \left( q(t) - \frac{p(t)}{R} - \left[ \sum_k \beta_k p^k \right] \cdot \left[ \dot{p}(t) - Z(p)\cdot(\dot{q}(t) - \dot{p}(t) - \dot{p}(t)/R) \right] \right)^2 dt$$

zu bestimmen und die individuelle Compliance-Funktion C(p) zu berechnen als

$$C(p) = \sum_k \beta_k p^k$$

11. Vorrichtung nach Anspruch 3 und 5, bei der der Computer ferner Mittel aufweist, die angepaßt sind, die für $p(t) \leq p$ (ts) berechnete Compliance-Funktion C(p) zu verwenden, um den Blutfluß q(t) nach einem vollständigen Funktionensystem zu entwickeln und q(t) in Form einer Fourierreihe gemäß folgender Gleichung zu beschreiben

$$q(t) = \sum_k q_k \sin\left( k\cdot\pi \cdot \frac{t - t_0}{t_s - t_0} \right)$$

wobei die Koeffizienten qk durch Minimierung des mittleren quadratischen Fehlers bestimmt werden und die Größen to und ts die Zeitpunkte des Öffnens und Schließens der Aortaklappe bezeichnen.

**12.** Vorrichtung nach irgendeinem der vorhergehenden Ansprüche, bei der der Computer ferner Mittel aufweist, die
angepaßt sind, den angenommenen Blutfluß q(t) derart zu variieren, dass der mittlere quadratische Fehler minimiert ist.

**13.** Vorrichtung nach irgendeinem der vorhergehenden Ansprüche, bei der der Computer ferner Mittel aufweist, die
angepaßt sind, das Aorta-Impedanz/Druck-Verhältnis durch

$$Z(p) = \frac{A}{\sqrt{C(p)}}$$

zu bestimmen, wobei A eine Proportionalitätskonstante ist.

**14.** Vorrichtung nach irgendeinem der vorhergehenden Ansprüche, bei der der Computer ferner Mittel aufweist, die
angepaßt sind, eine nichtlineare Aortenimpedanz-Funktion mit Hilfe der Fouriertransformierten von Blutdruck $\tilde{p}$ ($\omega$)
und Blutfluß $\tilde{q}$ ($\omega$) nach $1/Z = \tilde{q}$ ($\omega \rightarrow \infty$)/$\tilde{p}$ ($\omega \rightarrow \infty$)-1/R oder $Z$ ($p$) = $\tilde{p}$ ($\omega \rightarrow \infty$)/$\tilde{q}$ ($\omega \rightarrow \infty$) zu bestimmen.

**15.** Vorrichtung nach Anspruch 1, bei der der Computer ferner Mittel aufweist, die angepaßt sind, einen Blutfluß q(t)
auf der Grundlage des Druckes p(t) und der ersten zeitlichen Ableitung dp/dt zu bestimmen und die Impedanz-
Funktion

$$Z\left( p = \int_{-\Delta t}^{+\Delta t} p(t)dt / 2\Delta t \right)$$

nach

$$Z(p) = \sqrt{\left( \int_{t-\Delta t}^{t+\Delta t} p^2(t)dt - \left( \int_{t-\Delta t}^{t+\Delta t} p(t)dt \right)^2 \right) \Big/ \left( \int_{t-\Delta t}^{t+\Delta t} q^2(t)dt - \left( \int_{t-\Delta t}^{t+\Delta t} q(t)dt \right)^2 \right)}$$

zu berechnen.

**16.** Vorrichtung nach irgendeinem der vorhergehenden Ansprüche, bei der der Computer ferner Mittel aufweist, die
angepaßt sind, die Aortenimpedanz-Funktion Z(p) durch ein Polynom endlicher Ordnung zu approximieren und Z
(p) über den Druckbereich hinaus, der während der Kalibrierung aufgezeichnet wird, nach diesem Polynom zu
extrapolieren.

**17.** Vorrichtung nach Anspruch 1, die ferner Mittel aufweist, die angepaßt sind, den systemischen Blutfluß q(t) eines
Lebewesens kontinuierlich zu bestimmen, wobei die Vorrichtung ferner Computermittel aufweist, die angepaßt
sind, den Blutfluß q(t)
wenigstens aus $\dot{p}(t)$, p(t) und R unter Verwendung eines nichtlinearen modifizierten Windkessel-Modells zu berechnen.

**18.** Vorrichtung nach Anspruch 17, bei der die Computermittel ferner Mittel aufweisen, die angepaßt sind, den systemischen Blutdruck q(t) gemäß

$$q(t) = \frac{p(t)}{R} + C(p)[\dot{p}(t) - Z(p) \cdot (\dot{q}(t) - \dot{p}(t)/R)]$$

zu bestimmen.

**19.** Vorrichtung nach Anspruch 17, bei der die Computermittel ferner Mittel aufweisen, die angepaßt sind, das Schlagvolumen SV durch Integrieren des Flusses über einen Zeitraum gemäß

$$SV = \int q(t)dt$$

zu berechnen und wobei dieser Zeitraum dem Herzschlag oder der Dauer der Systole während des Herzschlags entspricht.

**20.** Vorrichtung nach den Ansprüchen 1 und 17, bei der die Computermittel ferner Mittel aufweisen, die angepaßt sind, das Schlagvolumen SV durch Vergleichen des kontinuierlichen Blutflusses q(t) mit einem Referenz-Herzzeitvolumen COref gemäß

$$SV = \gamma\int q(t)dt \qquad \text{mit} \qquad \gamma = \frac{COref}{HR\cdot\int q(t)dt}$$

zu berechnen.

**21.** Vorrichtung nach irgendeinem der vorhergehenden Ansprüche 16 und 17, bei der die Computermittel ferner Mittel aufweisen, die angepaßt sind, die Schlagvolumenvariation gemäß

$$SVV = \sqrt{\langle SV^2 \rangle - \langle SV \rangle^2}$$

zu berechnen, und ferner Mittel aufweisen, die angepaßt sind, den Schlagvolumenwert entsprechend dem Schlagvolumenvariationswert zu korrigieren, der allein oder in Kombination mit anderen Parametern verwendet wird, wie mittlerer Blutdruck MAP, systolischer Druck $AP_{SYS}$, diastolischer Druck $AP_{DIA}$ und Herzrate HR.

**22.** Vorrichtung nach irgendeinem der vorhergehenden Ansprüche, bei der die Computermittel ferner Mittel aufweisen, die angepaßt sind, das Herzzeitvolumen kontinuierlich zusammen mit der Herzrate HR aus dem Schlagvolumen SV nach CO = HR · SV zu berechnen.

**23.** Vorrichtung nach Anspruch 22, bei der die Computermittel ferner Mittel aufweisen, die angepaßt sind, den mittleren Druck MAP aus der Blutdruckkurve kontinuierlich zu bestimmen und den systemischen Widerstand kontinuierlich gemäß

$$R = \frac{MAP - CVP}{CO}$$

für den Wert des Zentralvenendrucks CVP, der gemessen oder geschätzt wurde, zu berechnen.

**24.** Vorrichtung nach Anspruch 22, bei der der Computer ferner Mittel aufweist, die angepaßt sind, den mittleren Druck MAP aus der Blutdruckkurve kontinuierlich zu bestimmen und die Compliance-Funktion kontinuierlich gemäß

$$C(p) = \frac{MAP^2 CO}{\langle p(t) \rangle} \cdot \frac{1}{3\cdot MAP\cdot p - 3\cdot MAP^2 - p^2}$$

zu berechnen.

**Revendications**

**1.** Dispositif pour une détermination individuelle in vivo de la fonction de compliance C(p) = dV/dp du système vasculaire en aval d'un ventricule d'un être vivant à partir de la pression sanguine p(t) et d'un débit cardiaque de référence COref, dans lequel ledit dispositif comprend :

a) des moyens de capteur de pression conçus pour déterminer continûment la pression p(t) dans l'aorte ou au voisinage de l'aorte ;
b) des moyens de calculateur comprenant

b1) des moyens conçus pour calculer la pression sanguine moyenne MAP à partir de la pression sanguine p(t) déterminée continûment,
b2) des moyens conçus pour calculer la résistance systémique R du corps comme étant

$$R = \frac{MAP - CVP}{COref}$$

CVP étant une pression veineuse centrale arbitraire qui est déterminée ou estimée, et COref étant une valeur de référence pour le débit cardiaque,
b3) des moyens pour calculer la dérivée première de la pression sanguine par rapport au temps p' (t) dp/dt, et
b4) des moyens pour calculer la fonction de compliance C(p) au moins à partir de p(t), p' (t) et R au moyen d'un modèle non linéaire de Wind-Kessel modifié.

2. Dispositif selon la revendication 1, dans lequel lesdites valeurs de p(t) sont des valeurs satisfaisant à la condition suivante pourcalculer la fonction de compliance C(p) :

$$p(t) \leq p(ts)$$

ts étant l'instant où la valve aortique se ferme.

3. Dispositif selon la revendication 1, dans lequel seulement des valeurs de pression sanguine relatives à la diastole sont utilisées dans le calcul de la fonction de compliance C(p).

4. Dispositif selon la revendication 1, dans lequel seulement des valeurs de pression sanguine provenant de la systole sont utilisées dans le calcul de la fonction de compliance C(p).

5. Dispositif selon l'une quelconque des revendications précédentes, dans lequel ledit calculateur comprend en outre des moyens conçus pour déterminer un débit sanguin q(t) sur la base de la pression p(t) et de la dérivée première par rapport au temps dp/dt, et calculer ladite fonction de compliance selon la relation

$$C(p) = \frac{q(t) - p(t)/R}{p'(t) - Z(p) \cdot (q'(t) - p'(t)/R)}$$

pour des fonctions arbitraires d'impédance Z(p) et des instants arbitraires t de sorte que la relation

$$q(t) = \frac{p(t)}{R} + C(p)[p'(t) - Z(p) \cdot (q'(t) - p'(t)/R)]$$

soit satisfaite.

6. Dispositif selon l'une quelconque des revendications précédentes, dans lequel ledit calculateur comprend en outre des moyens conçus pour déterminer de manière approchée l'inverse de la fonction d'élasticité, 1/C(p), au moyen d'un polynôme d'ordre fini, et pour extrapoler C(p) au-delà de la gamme de pression enregistrée quand ils déterminent le débit cardiaque de référence selon ledit polynôme.

7. Dispositif selon la revendication 1, dans lequel ledit calculateur comprend en outre des moyens conçus pour déterminer le minimum de la fonction

$$\Sigma \int_{t=t_o}^{t=t_s} \left( q(t) - \frac{p(t)}{R} - \frac{\dot{p}(t) - Z(p) \cdot (\dot{q}(t) - \dot{p}(t)/R)}{\Sigma_k \alpha_k p^k} \right)^2 dt$$

et pour calculer alors la fonction individuelle de compliance C(p) par la relation

$$C(p) = \frac{1}{\Sigma_k \alpha_k \, p^k}$$

**8.** Dispositif selon les revendications 3 et 6, dans lequel ledit calculateur comprend en outre des moyens conçus pour décrire l'inversé de la fonction d'élasticité au moyen d'un polynôme du deuxième ordre et déterminer C(p) de manière approchée par la fonction suivante

$$C(p) = \frac{MAP^2 \, COref}{< p'(t) >} \cdot \frac{1}{3 \cdot MAP \cdot p - 3 \cdot MAP^2 - p^2}$$

**9.** Dispositif selon l'une quelconque des revendications précédentes, dans lequel ledit calculateur comprend en outre des moyens conçus pour déterminer de manière approchée la fonction de compliance C(p) au moyen d'un polynôme d'ordre fini et extrapoler C(p) au-delà de la gamme de pression enregistrée quand il détermine le débit cardiaque de référence selon ledit polynôme.

**10.** Dispositif selon la revendication 1, dans lequel ledit calculateur comprend en outre des moyens conçus pour déterminer le minimum de la fonction

$$\Sigma \int_{t=t_o}^{t=t_s} \left( q(t) - \frac{p(t)}{R} - [\Sigma_k \beta_k p^k] \cdot [\dot{p}(t) - Z(p) \cdot (\dot{q}(t) - \dot{p}(t)/R] \right)^2 dt$$

et calculer la fonction individuelle d'élasticité C(p) par la relation

$$C(p) = \Sigma_k \beta_k p^k$$

**11.** Dispositif selon les revendications 3 et 5, dans lequel ledit calculateur comprend en outre des moyens conçus pour utiliser la fonction de compliance C(p) calculée pour $p(t) \leq p(ts)$ pour développer le débit sanguin q(t) sous la forme d'un système complet de fonctions et décrire q(t) sous la forme d'une série de Fourier selon l'équation suivante

$$q(t) = \sum_k q_k \sin\left( k \cdot \pi \cdot \frac{t - t_0}{t_s - t_0} \right)$$

les coefficients $q_k$ étant déterminés en minimisant l'erreur quadratique moyenne, et les valeurs t0 et ts désignant les instants où la valve aortique est ouverte et fermée.

**12.** Dispositif selon l'une quelconque des revendications précédentes, dans lequel ledit calculateur comprend en outre des moyens conçus pour faire varier le débit sanguin supposé q(t) de manière à minimiser l'erreur quadratique moyenne.

**13.** Dispositif selon l'une quelconque des revendications précédentes, dans lequel ledit calculateur comprend en outre des moyens conçus pour déterminer la relation entre impédance aortique et pression selon la relation

$$Z(p) = \frac{A}{\sqrt{C(p)}}$$

A étant une constante de proportionnalité.

**14.** Dispositif selon l'une quelconque des revendications précédentes, dans lequel ledit calculateur comprend en outre des moyens conçus pour déterminer une fonction impédance aortique non linéaire en utilisant les transformées de Fourier de la pression sanguine $\hat{p}(\omega)$ et du débit sanguin $\hat{q}(\omega)$ selon la relation

$$1/Z = \hat{q}(\omega \to \infty) / \tilde{p}(\omega \to \infty) - 1/R$$

ou

$$Z(p) = \tilde{p}(\omega \to \infty) / \tilde{q}(\omega \to \infty)$$

**15.** Dispositif selon la revendication 1, dans lequel ledit calculateur comprend en outre des moyens conçus pour déterminer un débit sanguin q(t) sur la base de la pression p(t) et de la dérivée première du temps dp/dt et calculer la fonction d'impédance

$$Z(p) = \int_{-\Delta t}^{+\Delta t} p(t)dt / 2\Delta t$$

en fonction de

$$Z(p) = \sqrt{\left(\int_{t-\Delta t}^{t+\Delta t} p^2(t)dt - \left(\int_{t-\Delta t}^{t+\Delta t} p(t)dt\right)^2\right) / \left(\int_{t-\Delta t}^{t+\Delta t} q^2(t)dt - \left(\int_{t-\Delta t}^{t+\Delta t} q(t)dt\right)^2\right)}$$

**16.** Dispositif selon l'une quelconque des revendications précédentes, dans lequel ledit calculateur comprend en outre des moyens Conçus pour déterminer de manière approchée la fonction d'impédance aortique Z(p) au moyen d'un polynôme d'ordre fini et extrapoler Z(p) selon ledit polynôme au-delà de la gamme de pression enregistrée pendant l'étalonnage.

**17.** Dispositif selon la revendication 1, comprenant en outre des moyens conçus pour déterminer continûment le débit sanguin systémique q(t) d'un être vivant, dans lequel ledit dispositif comprend en outre des moyens de calculateur conçus pour calculer ledit débit sanguin q(t) au moins à partir de p(t), p'(t) et R en utilisant un modèle non linéaire de Wind-Kessel modifié.

**18.** Dispositif selon la revendication 17, dans lequel lesdits moyens de calculateur comprennent en outre des moyens conçus pour déterminer ledit débit sanguin systémique q(t) selon la relation

$$q(t) = \frac{p(t)}{R} + C(p)[p'(t) - Z(p)\cdot(q'(t) - p(t)/R)]$$

**19.** Dispositif selon la revendication 17, dans lequel lesdits moyens de calculateur comprennent en outre des moyens conçus pour calculer le volume systolique SV en intégrant le débit sur une période de temps selon la relation

$$SV = \int q(t)dt$$

et dans lequel ladite période de temps correspond au battement cardiaque ou au temps d'éjection pendant le battement cardiaque.

20. Dispositif selon les revendications 1 et 17, dans lequel lesdits moyens de calculateur comprennent en outre des moyens conçus pour calculer le volume systolique SV en comparant le débit sanguin continu q(t) à un débit cardiaque de référence COref, selon la relation

$$SV = \gamma\int q(t)dt \qquad avec \qquad \gamma = \frac{COref}{HR \cdot \int q(t)dt}$$

21. Dispositif selon l'une quelconque des revendications 16 et 17, dans lequel lesdits moyens de calculateur comprennent en outre des moyens conçus pour calculer la variation de volume systolique selon la relation

$$SVV = \sqrt{\langle SV^2\rangle - \langle SV\rangle^2}$$

et des moyens conçus pour corriger la valeur de volume systolique en fonction de ladite valeur de variation de volume systolique prise isolément ou en combinaison avec d'autres pramètres tels que la pression sanguine moyenne MAP, la pression systolique $AP_{SYS}$, la pression diastolique $AP_{DIA}$ et le rythme cardiaque HR.

22. Dispositif selon l'une quelconque des revendications précédentes, dans lequel lesdits moyens de calculateur comprennent en ,outre des moyens conçus pour calculer en permanence le débit cardiaque en même temps que le rythme cardiaque HR à partir du volume systolique SV selon la relation CO = HR·SV.

23. Dispositif selon la revendication 23, dans lequel lesdits moyens de calculateur comprennent en outre des moyens conçus pour déterminer en permanence la pression moyenne MAP à partir de la courbe de pression sanguine et calculer en permanence la résistance systémique par la relation

$$R = \frac{MAP - CVP}{CO}$$

pour une valeur CVP de pression veineuse centrale qui a été mesurée ou estimée.

24. Dispositif selon la revendication 22, dans lequel lesdits moyens de calculateur comprennent en outre des moyens conçus pour déterminer en permanence la pression moyenne MAP à partir de la courbe de pression sanguine et calculer en permanence la fonction de compliance par la relation

$$C(p) = \frac{MAP^2\ CO}{\langle\dot{p}(t)\rangle} \cdot \frac{1}{3 \cdot MAP \cdot p - 3 \cdot MAP^2 - p^2}$$

Fig.1

Start

Calculate *MAD* and *HR* from *p(t)*.

Calculate the systemic resistance R from COa, MAP and where appropriate CVP

Initialize *q(t)* with the aid of COa and *p(t)*

Set $Z(p) = 0$, $q_{old}(t) = q(t)$ and $E_{old}=\infty$

Calculate *C(p)* according to Eq.(9)

Approximate $1/C(p)$ or $C(p)$, e. g. by a polynomial

Calculate the error *E*.

$E < E_{old}$ ?

No

Yes

Set $q_{old}(t) = q(t)$ and $E_{old}=E$.

Determine $Z(p)$ from $C(p)$, *q(t)* and *p(t)*.

Error *E* small enough ?

Yes

No

Set $q(t) = q_{old}(t) + \delta q(t)$.

End

Fig.2

21

Fig.3a

blood pressure p(t)
blood flow  q(t)

Fig.5